(19) Europäisches Patentamt
European Patent Office
Office européen des brevets

(11) Veröffentlichungsnummer: **0 000 542**
**B1**

(12) # EUROPÄISCHE PATENTSCHRIFT

(45) Veröffentlichungstag der Patentschrift: **15.07.81**

(21) Anmeldenummer: **78100436.1**

(22) Anmeldetag: **19.07.78**

(51) Int. Cl.³: **C 07 C 76/02,**
C 07 C 79/32,
C 07 C 79/35, C 07 C 79/46

(54) **Verfahren zur Herstellung von Trifluormethylphenolen.**

(30) Priorität: **26.07.77 DE 2733682**

(43) Veröffentlichungstag der Anmeldung:
**07.02.79 Patentblatt 79/3**

(45) Bekanntmachung des Hinweises auf die Patenterteilung:
**15.07.81 Patentblatt 81/28**

(84) Benannte Vertragsstaaten:
**BE CH DE FR GB NL**

(56) Entgegenhaltungen:
**US - A - 3 813 446**
**US - A - 3 943 180**

**JOURNAL OF ORGANIC CHEMISTRY, Vol. 36, Januar 15. 1971, Easton, R. L. JACOBS "The synthesis of nitrotrifluormethylphenols and related compounds from nitrotrifluoromethylchlorobenzenes" Seiten 242—243**

(73) Patentinhaber: **BAYER AG**
**Zentralbereich Patente, Marken und Lizenzen**
**D-5090 Leverkusen 1, Bayerwerk (DE)**

(72) Erfinder: **Marhold, Albrecht, Dr.**
**Carl-Duisberg-Strasse 329**
**D-5090 Leverkusen 1 (DE)**
Erfinder: **Klauke, Erich, Dr.**
**Eichendorffweg 8**
**D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 000 542 B1**

Courier Press, Leamington Spa, England.

Verfahren zur Herstellung von Trifluormethylphenolen

Die Erfindung betrifft ein Verfahren zur Herstellung von Phenolen mit einer Trifluormethylgruppe.

Es ist bereits bekannt geworden, daß sich zahlreiche Trifluormethyl-nitro-phenole durch Umsetzung von bestimmten halogenierten Nitrobenzotrifluoriden mit einer mindestens äquimolaren Menge an einem Alkalimetall- bzw. Erdalkalimetall-hydroxid in Gegenwart eines oder mehrerer Lösungsmittel aus der Gruppe von Dimethylsulfoxid, Dimethylformamid und Dimethylacetamid herstellen lassen (vgl. US—PS 3 813 446 und J. Org. Chem. *32*, 242 (1971)). Beispielsweise ist es möglich, 4-Chlor-3-nitrobenzotri-fluorid mit Natronlauge in Gegenwart von Dimethylsulfoxid in 2-Nitro-4-trifluormethyl-phenol zu überführen. Dieses bekannte Verfahren ist jedoch mit mehreren Nachteilen belastet. So erfordert es die Anwesenheit von Lösungsmitteln, die nicht nur teuer sind, sondern sich wegen ihres relativ hohen Siedepunktes vom gewünschten Endprodukt nur schwierig abtrennen lassen. Außerdem müssen bei der Verwendung von Dimethylsulfoxid, Dimethylformamid und/oder Dimethylacetamid als Lösungsmittel besondere Sicherheitsvorkehrungen getroffen werden, um Geruchsbelästigungen und Schädigungen durch diese verhältnismäßig stark toxischen Solventien zu vermeiden. Schließlich lassen die Ausbeuten bei diesem Verfahren häufig sehr zu wünschen übrig.

Nach einen anderen Verfahren erhält man Trifluormethylphenole durch Spaltung der entsprechenden Methoxyverbindungen mit Pyridin-hydrochlorid bei 210°C (J. org. Chem. *27*, 4660 (1962):bei diesem Verfahren werden die Methoxyverbindungen aus Chlornitrobenzotrifluoriden in einer vorgeschalteten Verfahrensstufe hergestellt.

Es wurde ein Verfahren zur Herstellung von Trifluormethylphenolen gefunden, bei dem man Verbindungen der Formel

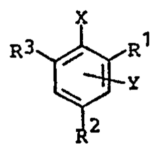

(I)

in der

X Halogen,

$R^1$, $R^2$ und $R^3$ Wasserstoff, Trifluormethyl, Nitro, Alkylsulfonyl, Arylsulfon, Carboxyl oder Carbonsäureester und

Y Wasserstoff, ein Halogen oder Alkoxy

bedeuten,

wobei von den Substituenten $R^1$, $R^2$ und $R^3$ einer eine Trifluormethylgruppe darstellt und mindestens ein weiterer von Wasserstoff verschieden ist, in alkoholischer Lösung mit überschüssigem wäßrigen Alkalihydroxid in Gegenwart von quartären Oniumsalzen umsetzt.

Halogene (X) können Fluor, Chlor, Brom oder Jod, bevorzugt Fluor oder Chlor, sein.

Als Substituenten $R^1$, $R^2$ oder $R^3$ seien neben Wasserstoff und Trifluormethyl beispielsweise genannt:

die Nitrogruppe, die Alkylsulfonylgruppe, deren Alkylrest bevorzugt ein geradkettiger oder verzweigter niederer Alkylrest mit 1—8 Kohlenstoffatomen sein kann, wie beispielsweise Methyl, Äthyl, Propyl, Isopropyl oder isomeres Butyl, Hexyl oder Octyl, die Arylsulfongruppe, deren Arylrest bevorzugt Phenyl oder ein beispielsweise durch Chlor oder Methyl substituiertes Phenyl sein Kann, die Carboxylgruppe, die Carbonsäureestergruppe, deren Alkoholkomponente ein geradkettiger oder verzweigter niederer aliphatischer Alkohol mit 1—8 Kohlenstoffatomen sein kann, wie beispielsweise Methanol, Äthanol, Propanol, Isopropanol, oder die isomeren Butanole, Hexanole oder Octanole.

Als Halogenatom (Y) seien beispielsweise Fluor, Chlor, Brom oder Jod, bevorzugt Chlor oder Brom, genannt.

Die Alkoxygruppe (Y) enthält bevorzugt Alkylreste mit 1 bis 4 Kohlenstoffatomen, beispielsweise den Methyl-, Äthyl-, Propyl-, Isopropyl-, Butyl- oder tert.-Butylrest.

Als bevorzugte Verbindungen der Formel (I) seien Verbindungen der Formel II

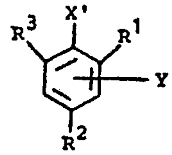

(II)

in der

X' ein Fluor- oder ein Chloratom bedeutet und die Reste $R^1$, $R^2$, $R^3$ die obengenannte Bedeutung haben, genannt.

Verbindungen der Formel I sind bekannt und können durch Nitrierung von Chlor-trifluormethylbenzol mit Salpetersäure/Schwefelsäure hergestellt werden (J. org. Chem. 26, 2707 (1961)).

Beispielsweise seien die folgenden Verbindungen der Formel (I) genannt:

4-Chlor-3-nitro-benzotrifluorid
2-Chlor-5-nitro-benzotrifluorid
3,4-Dichlor-5-nitro-benzotrifluorid
2,3-Dichlor-5-nitro-benzotrifluorid
2,4-Dichlor-5-nitro-benzotrifluorid
6-Chlor-3-nitro-5-trifluormethyl-benzoesäure
2-Chlor-3-nitro-5-trifluormethyl-benzoesäure
4-Brom-3-nitro-benzotrifluorid
2-Brom-5-nitro-benzotrifluorid
2-Chlor-3-methoxy-5-nitro-benzotrifluorid
2-Methoxy-4-chlor-5-nitro-benzotrifluorid.

Es ist als äußerst überraschend zu bezeichnen, daß sich aus den Verbindungen der Formel (I) die entsprechenden Trifluormethyl-phenole in glatter Reaktion und mit hoher Ausbeute herstellen lassen, denn auf Grund des bekannten Standes der Technik war anzunehmen, daß nicht nur der Substituent X gegen Hydroxy ausgetauscht werden würde, sondern auch eine Trifluormethylgruppe zu einer Carboxylgruppe verseift wird (vgl. J. Org. Chem. *26,* 2707 (1961)). Im Gegensatz su den Erwartungen ist nach dem erfindungsgemäßen Verfahren jedoch ein selektiver Halogen-Hydroxyl-Austausch ohne gleichzeitige Verseifung der Trifluormethylgruppe möglich.

Als Lösungsmittel für das erfindungsgemäße Verfahren können Alkonole mit einem niederen unverzweigten oder verzweigten Alkylrest eingesetzt werden. Bevorzugt seien Alkohole genannt, deren unverzweigter oder verzweigter Alkylrest 1 bis 4 Kohlenstoffatome hat, wie Methanol, Äthanol, Propanol, Isopropanol, die verschiedenen isomeren Butanole, Glykol und Glykolmonomethyläther.

Die Menge des Lösungsmittels kann in weiten Grenzen schwanken, so kann beispielsweise auf 1 Gew.-Teil eine Verbindung der Formel (I) 1 bis 20 Gew.-Teile, bevorzugt 2 bis 8 Gew.-Teile, Lösungsmittel eingesetzt werden.

Als Alkalihydroxide für das erfindungsgemäße Verfahren werden bevorzugt Natrium- oder Kaliumhydroxid eingesetzt. Im allgemeinen werden sie in Form ihrer wäßrigen Lösungen angewandt, die beispielsweise 30—50 Gew.-% Alkalihydroxid enthalten. Das Natrium- oder Kaliumhydroxid kann jedoch auch in fester Form in die wäßrige alkoholische Lösung eingetragen werden. Die Alkalihydroxide werden im allgemeinen im Überschuß angewendet, besonders vorteilhaft ist der Einsatz etwa der dreifachen molaren Menge bezogen auf die Umsetzung des Halogenatoms zur phenolischen Hydroxylgruppe.

Die Wassergehalt der Reaktionslösung kann in weiten Grenzen schwanken. Im allgemeinen kann er etwa 20 bis 25 Gew.-% betragen.

Für das erfindungsgemäße Verfahren können Oniumsalze der Formel

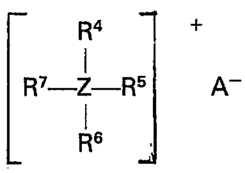

(III)

eingesetzt werden, in der

Z für ein Element der 5. Hauptgruppe des Periodensystems steht,

$R^4$ bis $R^7$ gleich oder verschieden sind und gegebenenfalls substituiertes Alkyl, Cycloalkyl, Aralkyl oder Aryl bedeuten oder zwei der Reste $R^4$ bis $R^7$ zusammen mit dem Zentralatom Z und gegebenenfalls weiteren Heteroatomen einen Heterocyclus bilden, wobei die Reste $R^4$ bis $R^7$ gemeinsam mindestens 6 Kohlenstoffatome enthalten, und

A eine unter den Bedingungen des erfindungsgemäßen Verfahrens inertes anorganisches oder organisches Anion bedeutet.

Als Elemente der 5. Hauptgruppe des Periodensystems (nach Mendelejeff) seien genannt: Stickstoff, Phosphor, Arsen, Antimon oder Wismut.

Die Reste $R^4$ bis $R^7$ können niedermolekulare oder hochmolekulare organische Reste sein.

Als niedermolekulare organische Reste seien gegebenenfalls substituierte Alkylreste, Cycloalkylreste, Aralkylreste oder Arylreste genannt. Zwei der Reste $R^4$ bis $R^7$ können auch zusammen mit dem Zentralatom Z und gegebenenfalls weiteren Heteroatomen einen Heterocyclus bilden.

Die gegebenenfalls substituierten oder verzweigten Alkylreste können vor allem solche mit 1—18

Kohlenstoffatomen, bevorzugt mit 1—12 Kohlenstoffatomen, sein, wie der Methyl-, Äthyl-, die isomeren Propyl-, Butyl-, Octyl-, Decyl-, oder Dodecylreste.

Die Cycloalkylreste können gegebenenfalls durch $C_1$—$C_4$-Alkylreste substituierte Cyclopentyl- und insbesondere Cyclohexylreste sein.

Die Aralkylreste können gegebenenfalls durch $C_1$—$C_4$-Alkylreste, Methoxygruppen oder Halogenatome substituierte Benzylreste sein.

Die Arylreste können gegebenenfalls durch $C_1$—$C_4$-Alkylreste, Methoxygruppen, Äthoxygruppen oder Halogenatome substituierte Phenylreste sein.

Bei einem Heterocyclus, den zwei benachbarte Reste $R^4$ bis $R^7$ gemeinsam mit dem Zentralatom Z und gegebenenfalls mit weiteren Heteroatomen bilden können, kommen als weitere Heteroatome bevorzugt Sauerstoff, Stickstoff oder Schwefel in Frage.

Bevorzugt seien die 5- und 6-gliedrigen Heterocyclen genannt, beispielsweise der Pyrrolidin-, der Piperidin oder der Morpholinring.

Als polymerer organischer Rest bei einem der Reste $R^4$ bis $R^7$ sei beispielsweise ein gegebenenfalls durch Divinylbenzol vernetztes Polystyrol genannt.

Die Gesamtzahl der Kohlenstoffatome aller Reste $R^4$ bis $R^7$ ist für das erfindungsgemäße Verfahren mindestens sechs. Die Höchstzahl aller Kohlenstoffatome kann beispielsweise bei einem polymeren organischen Rest sehr groß sein. Die bevorzugte Gesamtzahl der Kohlenstoffatome aller niedermolekularen Reste $R^4$ bis $R^7$ beträgt 8 bis 25.

Bevorzugte Anionen A sind das Chlorid-, Bromid- und Hydroxidanion.

Bevorzugte Oniumsalze für das erfindungsgemäße Verfahren sind quartäre Ammonium- oder Phosphoniumsalze der Formel

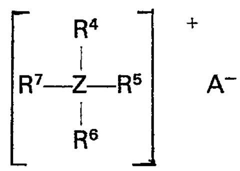

(IV)

in der

Z' Stickstoff oder Phosphor bedeutet und

$R^4$ bis $R^7$ und A die oben angegebene Bedeutung haben.

Beispiele für die bevorzugten quartären Ammonium- bzw. Phosphoniumsalze sind: Tetraäthylammoniumchlorid, Tetrabutylammoniumbromid, Träthyl-benzyl-ammoniumhydroxid, Benzyl-dimethyldodecyl-ammoniumchlorid, Dibutyl-piperidiniumbromid, Methyl-trioctylammoniumchlorid, Dimethylbenzyl-phenyl-ammoniumchlorid, Methyl-butyl-piperidiniumbromid, Benzyl-methyl-piperdiniumhydroxid, Triäthyl-phenyl-ammoniumchlorid, Dodecyl-trimethyl-ammoniumhydroxid, Cyclohexyldodecyl-dimethyl-ammoniumchlorid, Methyl-äthyl-piperidiniumbromid, Triphenyl-benzyl-phosphoniumbromid, Tetrabutyl-phosphoniumchlorid, Tributyl-benzyl-phosphoniumchlorid.

Quartäre Ammoniumverbindungen, bei denen einer der Reste $R^4$ bis $R^7$ ein polymerer Rest ist, können beispielsweise Ionenaustauscher auf der Basis von Styrol und im allgemeinen 2 Mol-% Divinylbenzol als Vernetzer, die quartäre Ammoniumgruppen tragen, sein.

Die eingesetzten Mengen des quartären Oniumsalzes können von 1 bis 20 Gew.-% schwanken und liegen im allgemeinen bei etwa 5 bis 10 Gew.-%, bezogen auf die Ausgansverbindung.

Die genannten quartären Oniumsalze sind als Phasenübergangskatalysatoren in Mehrphasensystemen bekannt und können nach bekannten Verfahren hergestellt werden (Houben-Weyl, 4. Aufl. Stuttgart 1958, Band XI, 2, S. 587 und Band XIV, 2. S. 750).

Das erfindungsgemäße Verfahren kann im Temperaturbereich von etwa 0 bis etwa 80°C, bevorzugt von etwa 50 bis 75°C, durchgeführt werden.

Das erfindungsgemäße Verfahren kann durch folgende Reaktionsgleichung erläutert werden:

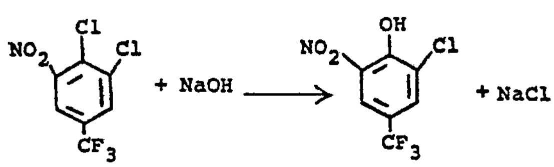


In einer besonderen Variante läßt sich das erfindungsgemäße Verfahren so durchführen, daß man

a) Verbindungen der Formel

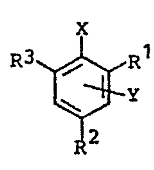

(I)

in welcher

X, Y, $R^1$, $R^2$ und $R^3$ die oben angegebene Bedeutung haben,

in Gegenwart eines als Lösungsmittel dienenden Alkohols mit einem niederen unverzweigten oder verzweigten Alkylrest mit überschüssigem, wäßrigen Alkalihydroxid in Gegenwart von quartären Oniumsalzen bei Temperaturen zwischen 15 und 35°C umsetzt und

b) die dabei entstehenden Verbindungen der Formel

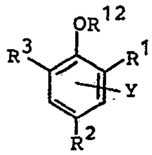

(V)

in welcher

Y, $R^1$, $R^2$ und $R^3$ die oben angegebene Bedeutung haben und

$R^{12}$ für einen niederen unverzweigten oder verzweigten Alkylrest steht,

in alkoholischer Lösung mit überschüssigem, wäßrigem Alkalihydroxid in Gegenwart von quartären Oniumsalzen umsetzt.

Die bei dieser Variante des erfindungsgemäßen Verfahrens als Zwischenprodukte auftretenden Alkoxyverbindungen der Formel

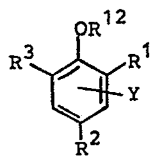

(V)

in der die Reste $R^1$, $R^2$, $R^3$ und Y die oben angegebene Bedeutung haben und

$R^{12}$ ein niederer unverzweigter oder verzweigter Alkylrest ist,

können isoliert werden, sofern man in dem Temperaturbereich zwischen 15 und 35°C arbeitet.

Die Alkylreste $R^{12}$ dieser Alkoxyverbindungen ist durch den als Lösungsmittel verwendeten Alkohol bestimmt; bei Verwendung von Methanol erhält man also eine methoxyverbindung.

Solche Alkoxyverbindungen sind bekannt (J. Org. Chem. *26,* 2707 (1961)) und können beispielsweise durch Reaktion eines organisch gebundenen Halogenatoms mit Natriummethylat oder mit methanolischem Kaliumhydroxid hergestellt werden.

Das erfindungsgemäße Verfahren kann beispielsweise wie folgt durchgeführt werden:

Im Reaktionsgefäß werden die Ausgangsverbindung, das Lösungsmittel und der Katalysator vorgelegt und unter Rühren das Alkalihydroxid, beispielsweise als wäßrige Lösung, zugetropft. Die Temperatur steigt dann an; man reguliert die Zutropfgeschwindigkeit so ein, daß die gewünschte Reaktionstemperatur nicht überschritten wird. Nach dem Ende der Zugabe wird bei der Reaktionstemperatur noch für einige Stunden nachgerührt. Das Phenol liegt in Form seines Alkalisalzes vor und kann in üblicher Weise durch Ansäuern mit einer Mineralsäure erhalten werden. Es kann nun durch Phasentrennung (bei Flüssigkeiten), durch Abfiltrieren (bei Festprodukten), durch Extraktion aus dem Reaktionsgemisch oder durch Wasserdampfdestillation abgetrennt werden.

Gegenüber den bekannten Verfahren zur Herstellung von Trifluormethylphenolen weist das erfindungsgemäße Verfahren den Vorteil auf, einfach durchführbar zu sein und in einer Stufe zu den gewünschten Phenolen zu führen.

Die quartären Oniumsalze der Formel (IV) sind als Phasenübergangskatalysatoren in Systemen mit zwei nicht miteinander mischbaren flüssigen Phasen bekannt (DT—OS 2 634 419).

Das erfindungsgemäße Verfahren wird dagegen mit quartären Oniumverbindungen in einem System mit nur einer flüssigen Phase durchgeführt.

Nach dem erfindungsgemäßen Verfahren können Trifluormethylphenole der Formel

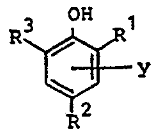

(VI)

in der die Reste $R^1$, $R^2$, $R^3$ und y die oben angegebene Bedeutung haben, hergestellt werden.

Die Trifluormethylphenole der Formel

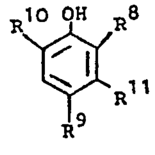

(VII)

in der

$R^8$ Trifluormethyl, Chlor oder Wasserstoff,
$R^9$ Trifluormethyl, Nitro oder Chlor,
$R^{10}$ Nitro, Chlor oder Carboxyl,
$R^{11}$ Methoxy oder Wasserstoff

'bedeuten,
wobei von den Substituenten $R^8$ und $R^9$ einer eine Trifluormethylgruppe darstellt und von den Substituenten $R^8$ und $R^{11}$ höchstens einer Wasserstoff ist, sind neu.

Beispielsweise seien als Verbindungen der Formel (VII) genannt:

2-chlor-4-trifluormethyl-6-nitro-phenol
2-Chlor-6-trifluormethyl-4-nitro-phenol
4-Chlor-2-trifluormethyl-6-nitro-phenol
3-Methoxy-4-trifluormethyl-6-nitro-phenol
5-Nitro-3-trifluormethyl-salicylsäure

Die Trifluormethylphenole, insbesondere die neuen Trifluormethylphenole, sind Zwischenprodukte zur Herstellung von Pflanzenschutzmitteln, insbesondere von Herbiziden (DT—OS 2 311 638, DT—OS 1 944 335).

Beispielsweise kann ein Trifluormethylphenol der Formel VI mit p-Nitrochlorbenzol in Gegenwart von Kali m-methylat zu Trifluormethyl-nitrodiphenyläther umgesetzt werden.

Herbicide aus neuen Trifluormethylphenolen der Formel VII zeigen verbesserte Wirkung gegenüber den bekannten Herbiciden.

## Beispiel 1

400 ml Methanol, 20 g Benzyldimethyldodecylammoniumchlorid und 180 g 4-Chlor-3-nitro-benzotrifluorid werden unter Rühren im Reaktionskolben vorgelegt und anschließend 400 g 50%ige Natronlauge zugetropft. Die Temperatur steigt bis ca. 70°C an, wenn die Zugabe in ca. 20 Minuten erfolgt. Es wird dann noch für 4 Stunden bei 65°C nachgerührt, anschließend durch Einwerfen von Eis abgekühlt und mit konzentrierter Salzsäure auf pH 3 gestellt. Das Phenol wird mit Wasserdampf übergetrieben, und die Phasen in der Vorlage durch Zugabe von ca. 50 ml konzentrierter Salzsäure getrennt.

Es fallen 156 g 2-Nitro-4-trifluormethylphenol mit einer Reinheit von mehr als 98,5% nach gaschromatographischer Analyse an, was einer Ausbeute von 91% der Theorie entspricht.
Siedepunkt: 92—95°C bei 15 mm,$n_D^{20}$: 1.5020.

## Beispiel 2

Man legt 450 g 2-chlor-5-nitro-benzotrifluorid in 1250 ml Methanol und 35 g Tetraäthylammoniumchlorid vor. Anschließend tropft man 1000 g einer wäßrigen, 50%igen Kalilauge zu (1 Std.), wobei die Temperatur durch Kühlen auf maximal 40°C gehalten wird. Etwa 30 Minuten nach Ende der Zugabe wird für 6 Stunden auf 80°C erhitzt, abgekühlt und mit konzentrierter Salzsäure sauer gestellt. Der Ansatz wird mehrmals mit je 300 ml Methylenchlorid extrahiert, die organische Phase mit Natriumsulfat getrocknet und unter vermindertem Druck vom Lösungsmittel befreit. Man erhält ca. 413 g Rohprodukt, F: 90—95°C mit einer Reinheit von ca. 95% nach gaschromatographischer Analyse. Nach Umkristallisation aus Toluol oder Destillation bei 0,5 mm fällt reines 4-Nitro-2-trifluormethylphenol an, F: 134°C.

## Beispiel 3

In 150 ml Methanol werden 50 g 3,4-Dichlor-5-nitro-benzotrifluorid und 5 g Benzyltriäthylammoniumbromid vorgelegt und bei 25 bis 30°C 100 g 50%ige Natronlauge zugetropft. Anschließend wird noch für 4 Stunden bei 65°C gerührt, abgekühlt, mit 100 ml Wasser versetzt und mit Schwefelsäure sauer gestellt. Das Rohprodukt wird mit Methylenchlorid extrahiert, die Lösung getrocknet und das Methylenchlorid abdestilliert. Der Rückstand wird fraktioniert destilliert, und man erhält 35 g 2-Chlor-4-trifluormethyl-6-nitrophenol mit einem Siedepunkt von 74 bis 76°C bei 0,25 mm.

F: 33—34°C aus Hexan. Ausbeute: 77% der Theorie.

## Beispiel 4

Im Reaktionsgefäß werden 50 g 2,3-Dichlor-5-nitro-benzotrifluorid in 150 ml Methanol und 5 g Tetrabutylammoniumchlorid vergelegt und anschließend 100 g 50%ige Natronlauge so zugetropft, daß die Temperatur auf maximal 50°C ansteigt. Bei dieser Temperatur wird noch für 5 Standen gerührt, dann abgekühlt und mit Salzsäure sauer gestellt. Das Produkt wird in Methylenchlorid aufgenommen und nach dem Trocknen mit Natriumsulfat destilliert. Bei $K_{P_{0,1}}$:110 bis 115°C gehen 33 g 2-Chlor-6-trifluormethyl-4-nitrophenol über, das einen Schmelzpunkt von 78 bis 80°C hat.

Ausbeute: 73% der Theorie.

Beispiel 5

In 400 ml Methanol werden 104 g 2,4-Dichlor-5-nitrobenzotrifluorid und 7 g Tetrabutylphosphoniumchlorid vorgelegt und 400 g 50%ige Natronlauge zugetropft. Die Temperatur wird auf maximal 70°C gehalten und nach Ende des Zutropfens noch für 6 Stunden nachgerührt. Der abgekühlte Ansatz wird mit Salzsäure sauer gestellt, anschließend das Produkt mit Methylenchlorid extrahiert. Nach dem Abdestillieren des Lösungsmittels wird der Rückstand aus Isopropanol umkristallisiert. Man erhält 69 g 3-Methoxy-4-trifluormethyl-6-nitrophenol mit einem Schmelzpunkt von 77 bis 78°C, die Ausbeute beträgt 73% der Theorie.

Beispiel 6

In 150 ml Äthylenglykolmonomethyläther werden 45 g 4-Chlor-3-nitro-benzotrifluorid und 10 ml einer 50%igen, wäßrigen Benzyldodecyldimethylammoniumchlorid vorgelegt und 100 g 50%ige Kalilauge in ca. 30 Minuten bis zu einer Temperatur von 65°C zugetropft. Dann rührt man für 6 Stunden bei 65°C, kühlt anschließend ab, stellt mit konzentrierter Salzsäure und treibt mit Wasserdampf ca. 500 ml Destillat über. Die organische Phase wird abgetrennt, mit 20 ml verdünnter Salzsäure gewaschen, erneut abgetrennt und mit Natriumsulfat getrocknet. Man erhält 38 g 2-Nitro-4-trifluormethylphenol, was einer Ausbeute von 92% der Theorie entspricht.

Beispiel 7

Wiederholt man Beispiel 6 und verwendet 150 ml Äthanol anstatt Äthylenglykolmonomethyläther, so erhält man 39 g 2-Nitro-4-trifluormethylphenol, das entspricht 94% der Theorie.

Beispiel 8

Man löst 27 g 6-Chlor-3-nitro-5-trifluormethylbenzosäure in 100 ml Methanol, fügt 1 g Trioctylmethyl-ammoniumchlorid hinzu und tropft dann unter Rühren 70 g 50%ige Natronlauge hinzu, wobei die Temperatur nicht über 65°C steigen soll. Nachdem man für weitere 4 Stunden bei 65°C gerührt hat, kühlt man ab und fügt so viel Wasser hinzu, bis alles in Lösung gegangen ist. Dann wird durch Zutropfen von konzentrierter Salzsäure bei 20 bis 30°C sauer eingestellt, worauf das Produkt ausfällt und abgesaugt wird. Die rohe 5-Nitro-3-trifluormethyl-salicylsäure wird aus Toluol umkristallisiert, F: 171—173°C, Ausbeute: 18 g; das entspricht 72% der Theorie.

Beispiel 9

In 200 ml Methanol werden 27 g 2-Chlor-3-nitro-5-trifluormethyl-benzosäure und 1 g Tetraäthylammoniumchlorid vorgelegt und unter Rühren 70 g 50%ige Natronlauge zugetropft. Man rührt für 5 Stunden bein 65°C nach, verdünnt mit 300 ml $H_2O$ und stellt anschließend mit Salzsäure sauer. Das Festprodukt wird abgesaugt und aus Wasser umkristallisiert. Man erhält 16 g 2-Hydroxy-3-nitro-5-trifluormethyl-benzoesäure mit dem Schmelzpunkt: 166 bis 168°C.

Beispiel 10

45 g 4-Chlor-3-nitro-benzotrifluorid werden in 150 ml Methanol gelöst vorgelegt. Dann trägt man 80 g Ionenaustauscher-Harz mit quartären Ammoniumgruppen ein, rührt für 10 Minuten, tropft 100 g 50%ige Natronlauge bis zu einer maximalen Temperatur von 70°C zu und rührt für 8 Stunden bei 65°C. Nach Abkühlen des Ansatzes wird mit konzentrierter Salzsäure angesäuert, vom Ionenaustauscher abfiltriert und das Rohrprodukt mit Wasserdampf destilliert. Man erhält 27 g 2-Nitro-4-trifluormethylphenol. $n_D^{20}$: 1.5003.

Beispiel 11

A) Man legt 45 g Natriumhydroxid und 10 g Tetraäthylammoniumchlorid in 200 g Methanol vor und tropft bei 25 bis 30°C 112,5 g 2-Chlor-5-nitro-benzotrifluorid zu, anschließend rührt man noch für 5 Stunden bei 30°C. Dann gießt man den Ansatz in 250 ml Wasser und extrahiert mit Methylenchlorid. Nach dem Verdampfen des Lösungsmittels kristallisiert man die Verbindung aus Isopropanol um. Man erhält 67 g 2-Methoxy-5-nitro-benzotrifluorid mit dem Schmelzpunkt 70 bis 74°C.

B) 67 g 2-Methoxy-5-nitro-benzotrifluorid werden in 150 ml Methanol gelöst, zuerst 8 g Tetraäthylammoniumchlorid zugegeben und dann 150 g 50%ige Kalilauge bis zu einer Temperatur von 70°C zugetropft. Man rührt bei 70°C noch für 8 Stunden nach, kühlt ab, stellt sauer und extrahiert mit Methylenchlorid. Nach dem Verdampfen des Methylenchlorids verbleiben 52 g rohes 4-Nitro-2-trifluormethyl-phenol, F: 90 bis 94°C, das durch Destillation gereinigt werden kann.

**Patentansprüche**

1. Verfahren zur Herstellung von Trifluormethylphenolen, dadurch gekennzeichnet, daß man Verbindungen der Formel

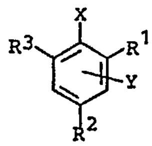

in der

X  Halogen,

$R^1$, $R^2$ und $R^3$ Wasserstoff, Trifluormethyl, Nitro, Alkylsulfonyl, Arylsulfon, Carboxyl oder Carbonsäureester und

Y  Wasserstoff, ein Halogen oder Alkoxy

bedeuten,

wobei von den Substituenten $R^1$, $R^2$ und $R^3$ einer eine Trifluormethylgruppe darstellt und mindestens ein weiterer von Wasserstoff verschieden ist, in alkoholischer Lösung mit überschüssigem wäßrigen Alkalihydroxid in Gegenwart von quartären Oniumsalzen umsetzt.

2. Verfahren nach Anspruch 1, dadurch gekennzeichnet, daß man in Gegenwart eines quartären Oniumsalzes der Formel

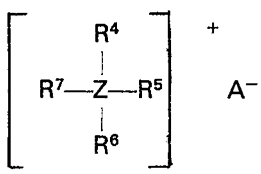

in der

Z  ein Atom der fünften Hauptgruppe des Periodensystems

$R^4$ bis $R^7$ unabhängig voneinander gegebenenfalls substituiertes Alkyl, Cycloalkyl, Aralkyl oder Aryl bedeuten oder zwei der Reste $R^4$ bis $R^7$ zusammen mit dem Zentralatom Z und gegebenenfalls weiteren Heteroatomen einen heterocyclus bilden, wobei die Reste $R^4$ bis $R^7$ gemeinsam mindestens 6 Kohlenstoffatome enthalten, und

A  ein Chlorid-, Bromid- oder Hydroxidanion bedeuten; arbeitet.

3. Verfahren nach Anspruch 1 und 2, dadurch gekennzeichnet, daß das quartäre Oniumsalz ein quartäres Ammonium- oder Phosphoniumsalz ist, dessen Substituenten insgesamt 8 bis 25 Kohlenstoffatome enthalten.

4. Verfahren nach Anspruch 1 und 2, dadurch gekennzeichnet, daß man als quartäres Ammoniumsalz einen Ionenaustauscher auf der Basis von Styrol mit 2 Mol-% Divinylbenzol als Vernetzer, der quartäre Ammoniumgruppen enthält, einsetzt.

5. Verfahren nach Anspruch 1 bis 4, dadurch gekennzeichnet, daß das quartäre Oniumsalz in einer Menge von 1 bis 20 Gew.-%, bezogen auf das Halogenbenzotrifluorid, eingesetzt wird.

6. Verfahren nach Anspruch 1 bis 5, dadurch gekennzeichnet, daß das quartäre Oniumsalz in einer Menge von 5 bis 10 Gew.-%, bezogen auf das Halogenbenzotrifluorid eingesetzt wird.

7. Verfahren nach Anspruch 1 bis 6, dadurch gekennzeichnet, daß man als alkoholisches Lösungsmittel Alkohole mit einem niederen unverzweigten oder verzweigten Alkylrest einsetzt.

8. Verfahren nach Anspruch 1 bis 7, dadurch gekennzeichnet, daß man das Alkalihydroxid in einer dreifach molaren Menge, bezogen auf das Halogenbenzotrifluorid, einsetzt.

9. Verfahren nach Anspruch 1 bis 8, dadurch gekennzeichnet, daß man als Alkalihydroxid Natriumhydroxid oder Kaliumhydroxid einsetzt.

10. Verfahren nach Anspruch 1 bis 9, dadurch gekennzeichnet, daß man die Reaktion im Temperaturbereich von 0 bis 80°C durchführt.

11. Verfahren nach Anspruch 1, dadurch gekennzeichnet, daß man

a) Verbindungen der Formel

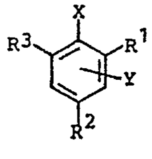

(I)

in welcher

X, Y, $R^1$, $R^2$ und $R^3$ die oben angegebene Bedeutung haben,

in Gegenwart eines als Lösungsmittel dienenden Alkohols mit einem niederen unverweigten oder verzweigten Alkylrest mit überschüssigem, wäßrigen Alkalihydroxid in Gegenwart von quartären Oniumsalzen bei Temperaturen zwischen 15 und 35°C umsetzt und

b) die dabei entstehenden Verbindungen der Formel

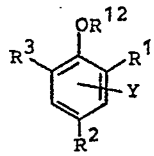

(V)

in welcher

Y, $R^1$, $R^2$ und $R^3$ die oben angegebene Bedeutung haben und

$R^{12}$ für einen niederen unverzweigten oder verzweigten Alkylrest steht,

in alkoholischer Lösung mit überschüssigem, wäßrigem Alkalihydroxid in Gegenwart von quartären Oniumsalzen umsetzt.

## Claims

1. Process for the preparation of trifluoromethylphenols, characterised in that compounds of the formula

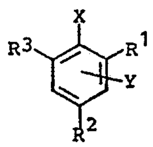

in which

X denotes halogen,

$R^1$, $R^2$ and $R^3$ denote hydrogen, trifluoromethyl, nitro, alkylsulphonyl, arylsulphone, carboxyl or carboxylic acid ester and

Y denotes hydrogen, a halogen or alkoxy, one of the substituents $R^1$, $R^2$ and $R^3$ representing a trifluoromethyl group and at least one other of them being different from hydrogen,

are reacted with excess aqueous alkali metal hydroxide in alcoholic solution in the presence of quaternary onium salts.

2. Process according to Claim 1, characterised in that the reaction is carried out in the presence of a quaternary onium salt of the formula

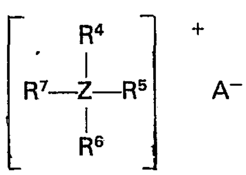

in which

Z denotes an atom of main group 5 of the periodic system,

$R^4$ to $R^7$ independently of one another denote optionally substituted alkyl, cycloalkyl, aralkyl or aryl, or two of the radical $R^4$ to $R^7$, together with the central atom Z and optionally further hetero-atoms, form a heterocyclic ring, the radicals $R^4$ to $R^7$ together containing at least 6 carbon atoms, and

A denotes a chloride, bromide or hydroxide anion.

3. Process according to Claim 1 and 2, characterised in that the quaternary onium salt is a quaternary ammonium or phosphonium salt, the substituents of which contain a total of 8 to 25 carbon atoms.

4. Process according to Claim 1 and 2, characterised in that an ion exchanger which is based on styrene with 2 mol% of divinylbenzene as a cross-linking agent and which contains quaternary ammonium groups is employed as the quaternary ammonium salt.

5. Process according to Claim 1 to 4, characterised in that the quaternary onium salt is employed in an amount of 1 to 20% by weight, relative to the halogenobenzotrifluoride.

6. Process according to Claim 1 to 5, characterised in that the quaternary onium salt is employed in an amount of 5 to 10% by weight, relative to the halogenobenzotrifluoride.

7. Process according to Claim 1 to 6, characterised in that alcohols with a lower unbranched or branched alkyl radical are employed as the alcoholic solvent.

8. Process according to Claim 1 to 7, characterised in that the alkali metal hydroxide is employed in three times the molar amount, relative to the halogenobenzotrifluoride.

9. Process according to Claim 1 to 8, characterised in that sodium hydroxide or potassium hydroxide is employed as the alkali metal hydroxide.

10. Process according to Claim 1 to 9, characterised in that the reaction is carried out in the temperature range from 0 to 80°C.

11. Process according to Claim 1, characterised in that

a) compounds of the formula

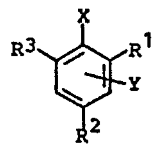

(I)

in which

X, Y, $R^1$, $R^2$ and $R^3$ have the meaning indicated above are reacted with excess aqueous alkali metal hydroxide in the presence of an alcohol with a lower unbranched or branched alkyl radical acting as solvent, in the presence of quaternary onium salts at temperatures between 15° and 35°C and

b) the resulting compounds of the formula

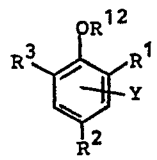

(V)

in which

Y, $R^1$, $R^2$ and $R^3$ have the abovementioned meaning and

$R^{12}$ represents a lower unbranched or branched alkyl radical,

are reacted with excess aqueous alkali metal hydroxide in alcoholic solution in the presence of quaternary onium salts.

**Revendications**

1. Procédé de production de trifluorométhylphénols, caractérisé en ce qu'on fait réagir des composés de formule:

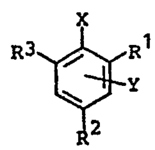

dans laquelle:

X  est un halogène,

$R^1$, $R^2$ $R^3$ représentent de l'hydrogène ou un groupe trifluorométhyle, nitro, alkylsulfonyle, arylsulfone, carboxyle ou ester d'acide carboxylique, et

Y  est de l'hydrogène, un halogène ou un groupe alkoxy,

l'un des substituants $R^1$, $R^2$ et $R^3$ représentant un groupe trifluorométhyle et au moins un autre étant différent d'un atome d'hydrogène, en solution alcoolique avec un hydroxyde alcalin aqueux en excès en présence de sels d'onium quaternaire.

2. Procédé suivant la revendication 1, caractérisé en ce qu'on opère en présence d'un sel d'onium quaternaire de formule:

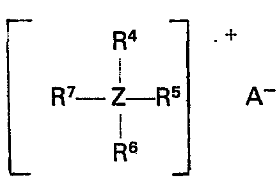

dans laquelle:

Z  est un atome du cinquième groupe du Système Périodique,

$R^4$ à $R^7$ représentent, indépendamment l'un de l'autre, un groupe alkyle, cyclo-alkyle, aralkyle ou aryle éventuellement substitué ou deux des restes $R^4$ à $R^7$ forment un hétérocycle avec l'atome central Z et, le cas échéant, avec d'autres hétéro-atomes, les restes $R^4$ à $R^7$ comprenant ensemble au moins 6 atomes de carbone, et

A  est un anion chlorure, bromure ou hydroxyde.

3. Procédé suivant l'une des revendications 1 et 2, caractérisé en ce que le sel d'onium quaternaire est un sel d'ammonium ou phosphonium quaternaire dont les substituants totalisent 8 à 25 atomes de carbone.

4. Procédé suivant l'une des revendications 1 et 2, caractérisé en ce qu'on utilise comme sel d'ammonium quaternaire un échangeur d'ions à base de styrène avec 2 moles % de divinylbenzène comme agent de réticulation, qui porte des groupes ammonium quaternaire.

5. Procédé suivant les revendications 1 à 4, caractérisé en ce qu'on utilise le sel d'onium quaternaire en une quantité de 1 à 20% en poids par rapport à l'halogénobenzotrifluorure.

6. Procédé suivant les revendications 1 à 5, caractérisé en ce que le sel d'onium quaternaire est utilisé en une quantité de 5 à 10% en poids par rapport à l'halogénobenzotrifluorure.

7. Procédé suivant les revendications 1 à 6, caractérisé en ce qu'on utilise comme solvant alcoolique des alcools portant un reste alkyle inférieur non ramifié ou ramifié.

8. Procédé suivant les revendications 1 à 7, caractérisé en ce qu'on utilise l'hydroxyde de métal alcalin en une quantité molaire triple par rapport à l'halogénobenzotrifluorure.

9. Procédé suivant les revendications 1 à 8, caractérisé en ce qu'on utilise comme hydroxyde de métal alcalin l'hydroxyde de sodium ou l'hydroxyde de potassium.

10. Procédé suivant les revendications 1 à 9, caractérisé en ce qu'on conduit la réaction dans la plage de températures de 0 à 80°C.

11. Procédé suivant la revendication 1, caractérisé en ce que,

a) on fait réagir des composés de formule:

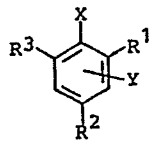

(I)

dans laquelle:
X, Y, $R^1$, $R^2$ et $R^3$ ont la définition donnée ci-dessus, en présence d'un alcool portant un reste alkyle inférieur non ramifié ou ramifié, servant de solvant, avec un hydroxyde alcalin aqueux en excès en présence de sels d'onium quaternaire à des températures comprises entre 15 et 35°C, et

b) on fait réagir les composés ainsi produits, de formule:

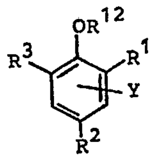

(V)

dans laquelle:
Y, $R^1$, $R^2$ et $R^3$ ont la définition donnée ci-dessus, et
$R^{12}$ désigne un reste alkyle inférieur non ramifié ou ramifié,
en solution alcoolique avec un hydroxyde alcalin aqueux en excès en présence de sels d'onium quaternaire.